# EUROPEAN PATENT APPLICATION

(11) **EP 1 568 358 A1**
(43) Date of publication of application: **31.08.2005**
(21) Application number: 04075593.6
(22) Date of filing: 27.02.2004
(51) Int. Cl.: A61K 7/50

(54) **Method for manufacturing a solid effervescent bath product**

(71) Applicant: sa Cardiff nv, 9870 Zulte (BE)
(72) Inventor: Goethals, Christophe, 9870 Zulte (BE)
(74) Representative: Quintelier, Claude

(57) **Abstract**

A method for manufacturing a cosmetic product, in particular a cosmetic product intended to be used in a bath, said method comprises mixing together cosmetic oil and a solvent, in order to obtain a cosmetic mixture, whereby sodium bicarbonate and citric acid are added to said cosmetic mixture in such an amount that the sodium bicarbonate forms between 35 % and 40 % of the total weight of said cosmetic mixture and the citric acid forms between 18 % and 20 % of said total weight, said cosmetic mixture being poured into a mould and an object is inserted into said cosmetic mixture in such a manner as to hide said object into said cosmetic mixture present in said mould, said cosmetic mixture being thereafter compressed within said mould with a force of at least 7 N in order to provide a solid structure to said cosmetic mixture and form with said cosmetic product a predetermined shape.

## Description

The present invention relates to a method for manufacturing a cosmetic product, in particular a cosmetic product intended to be used in a bath, said method comprises mixing together cosmetic oil and a solvent, in order to obtain a cosmetic mixture.

Such a method is generally known for the manufacturing of cosmetic products to be used in a bath or shower. The oil is used to flavour the product and the solvent to make the cosmetic product water soluble. Such cosmetic products are presented in different shapes and with a variety of colours and flavours in order to make the product attractive for the customer.

Manufacturers of cosmetic products are faced with a problem of making their products more attractive than those of their competitors, this however without making them much more expensive or without making a sacrifice with respect to the quality and the properties of the cosmetic product.

It is therefor an object of the present invention to present a method for manufacturing a cosmetic product which is more attractive without reducing the quality of the product and still maintain an economically attractive method.

For this purpose, a method according to the invention is characterised in that sodium bicarbonate and citric acid are added to said cosmetic mixture in such an amount that the sodium bicarbonate forms between 35 % and 40 % of the total weight of said cosmetic mixture and the citric acid forms between 18 % and 20 % of said total weight, said cosmetic mixture being poured into a mould and an object is inserted into said cosmetic mixture in such a manner as to hide said object into said cosmetic mixture present in said mould, said cosmetic mixture being thereafter compressed within said mould with a force of at least 7 N in order to provide a solid structure to said cosmetic mixture and form with said cosmetic product a predetermined shape. The addition of sodium bicarbonate and citric acid causes the obtained product to quickly dissolve while obtaining a relatively hard structure upon manufacturing. By compressing the mixture in a mould, a relatively solid product is obtained which will not break if it would by accident fall on the ground. The presence of an object hidden into the mixture before the latter is compressed, enables to hide the object in the final cosmetic product, which object will only become visible after the product has dissolved in the water. The presence of this hidden object makes the cosmetic product more attractive as it enables to provide a surprising effect to the end user.

A first preferred embodiment of a method according to the present invention is characterised in that an on water floatable object is inserted as said object. In such a manner, the hidden object will appear on the surface of the water, avoiding thereby that the end user has to look for it.

A second preferred embodiment of a method according to the present invention is characterised in that sodium carboxymethylcellulose (CMC) in an amount between 1 % and 1,5 % of said total weight is added to said cosmetic mixture. The use of CMC hardens the structure of the cosmetic product.

Preferably an amount between 0,03 % and 0,08 % of said total weight is added as a colour compound to said cosmetic mixture, said colour compound comprises C.I. 17200, C.I. 42090 and C.I. 19140 as colour substance. A coloured shape can in such a manner be given to the cosmetic product, without leaving stains on the body of the end user or on the bathroom furniture.

A third preferred embodiment of a method according to the present invention is characterised in that said cosmetic product is packed in a box comprising a label identifying said object. In such a manner the person buying the cosmetic product can select by using the label which surprise he or she will give to someone.

The invention will now be described in more details with reference to the drawings illustrating a preferred embodiment of a cosmetic product obtained by applying the method according to the present invention.

In the drawings :
figure 1 shows a ball shaped cosmetic product; and
figure 2 shows a cross section along the line II - II" in figure 1.

In the drawings a same reference sign has been allotted to a same or analogous element.

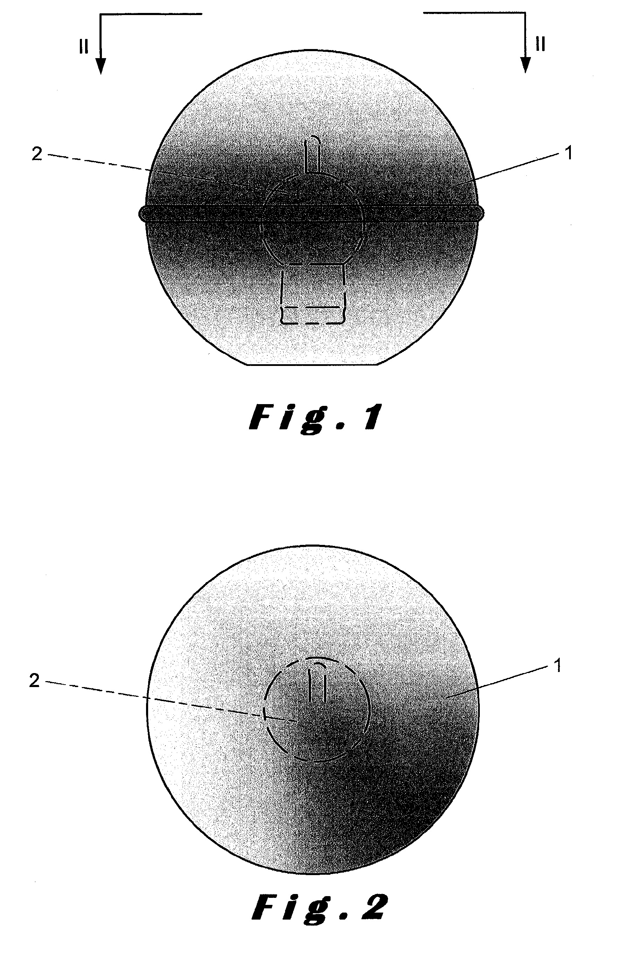

The cosmetic product 1 obtained by application of the method according to the present invention and illustrated in figure 1, has a ball shaped geometrical shape. Of course the cosmetic product can have whatever geometrical three-dimensional shape such as cylindrical, triangular, squared, hexagonal or arbitrary. The final shape of the product will essentially be determined by the costs of the mould necessary to provide the final shape.

Inside the cosmetic product 1 an object 2 is hidden. As the object is completely wrapped in the material forming the cosmetic product as such, it is not necessary that the object is placed in the centre of the cosmetic product as long as it is covered by the material of the cosmetic product and can not be seen from the outside.

Preferably, the cosmetic product has a width which does not exceed 10 cm in order to easily handle it. The hidden object can be of any shape and any kind, provided that it can be hidden inside the final product. So for example, the object can be a toy, which is of particular interest for children. The object could also be a sweet or a tablet placed in a container or in a paper. A message printed, with a water resistant ink on a water resistant carrier such as nylon or a plastic material, could also form the hidden object. The message could have a funny contents or be a love message.

For manufacturing the cosmetic product, a cosmetic mixture is made comprising cosmetic oil and a solvent. The solvent of course is necessary for dissolving the product, whereas the oil gives the flavour and smell. The cosmetic oil preferably forms 0,5 % to 0,9 % of the total weight of the cosmetic mixture. 0,03 % to 0,08 % of a colour compound could also be present in order to colour the water of the bath in which the product will be dropped. Care has however to be taken that the colour compound does not leave stains on the body of the end user on the bathroom furniture. For that purpose, the colour compound should comprise C.I. 17200 (pink), Cl 42090 (blue) and/or C.I. 19140 (yellow), where C.I. stands for colour identification number. Preferably an amount situated between 28 % en 32 % of sodium sulphate is added to the cosmetic mixture in order to obtain a cleaning effect.

Furthermore 35 % to 40 % of a total weight of the cosmetic mixture is formed by sodium bicarbonate and 18 % to 20 % of the total weight of the cosmetic mixture is formed by citric acid. Those components make that the mixture quickly dissolves in water, namely in a time period of 5 to 900 seconds. That the mixture quickly dissolves is of importance as the end user does not want to wait a long time before the surprising object appears. Preferably an amount of 1 % to 1,5 % sodium carboxymethyl-cellulose (CMC) is also present in the mixture in order to render the final product sufficiently hard. If the product is not hard enough, it could break if it would by accident fall on the ground, thereby undoing the surprising effect. Finally it is preferable to add between 3 % and 4 % of polyethylene glycol (PE 675) in order to soften the bath water.

Once the cosmetic mixture has been prepared, the latter is poured into a mould in order to shape the product to be obtained. The object can be entered simultaneously with the pouring of the mixture or after having poured the mixture into the mould. Care has however to be taken that the object is hidden in the mixture before compressing the latter. The latter is indeed compressed in the mould with a force of 7 to 8 Newton in order to make the structure hard enough and to provide the final shape.

After manufacturing, the product is packed. The package could be formed by a simple foil or could be formed by a box. The package is preferably provided with a label which is in a removable way adhered or attached thereon. The label comprises a definition of the object hidden inside the cosmetic product. In such a manner, the consumer who wants to surprise a relative can select what kind of surprise he or she would like to give. By reading the contents of the label, the hidden object can be defined. So, for example if the object consists of a love message, the consumer can select what love message he or she will offer. The fact that the label is adhered in a removable way, makes it possible to remove the label before giving the cosmetic product to an end user.

## Claims

1. A method for manufacturing a cosmetic product, in particular a cosmetic product intended to be used in a bath, said method comprises mixing together cosmetic oil and a solvent, in order to obtain a cosmetic mixture, **characterised in that** sodium bicarbonate and citric acid are added to said cosmetic mixture in such an amount that the sodium bicarbonate forms between 35 % and 40 % of the total weight of said cosmetic mixture and the citric acid forms between 18 % and 20 % of said total weight, said cosmetic mixture being poured into a mould and an object is inserted into said cosmetic mixture in such a manner as to hide said object into said cosmetic mixture present in said mould, said cosmetic mixture being thereafter compressed within said mould with a force of at least 7 N in order to provide a solid structure to said cosmetic mixture and form with said cosmetic product a predetermined shape.

2. A method as claimed in claim 1, **characterised in that** an on water floatable object is inserted as said object.

3. A method as claimed in claim 1 or 2, **characterised in that** sodium carboxymethylcellulose (CMC) in an amount between 1 % and 1,5 % of said total weight is added to said cosmetic mixture.

4. A method as claimed in claim 1, 2 or 3, **characterised in that** an amount between 0,5 % and 0,9 % of said total weight is introduced as cosmetic oil into said cosmetic mixture.

5. A method as claimed in any one of the claims 1 to 4, **characterised in that** an amount between 0,03 % and 0,08 % of said total weight is added as a colour compound to said cosmetic mixture, said colour compound comprises C.I. 17200, C.I. 42090 and C.I. 19140 as colour substance.

6. A method as claimed in any one of the claims 1 to 5, **characterised in that** an amount between 28 % and 32 % of sodium sulphate is added to said cosmetic mixture.

7. A method as claimed in any one of the claims 1 to 6, **characterised in that** an amount between 3 % and 4 % of polyethylene glycol is added to said cosmetic mixture.

8. A method as claimed in any one of the claims 1 to 7, **characterised in that** said cosmetic product is packed in a box comprising a label identifying said object.

9. A method as claimed in claim 8, **characterised in that** said label is adhered to said box in a removable way.

10. A method as claimed in any one of the claims 1 to 9, **characterised in that** the introduced object is formed by a message printed on a carrier with a water resistant ink.
